# EUROPEAN PATENT APPLICATION

(11) **EP 4 491 102 A1**
(43) Date of publication of application: **15.01.2025**
(21) Application number: 23185656.8
(22) Date of filing: 14.07.2023
(51) Int. Cl.: A61B 5/00, A61B 5/1455, A61B 5/145

(54) **MONITORING DEVICE FOR MONITORING OXYGENATION OF CEREBRAL TISSUE**

(71) Applicant: Universität Zürich, 8006 Zürich (CH)
(72) Inventor: Kayanov, Alexander, 8006 Zürich (CH); Jiang, Jingjing, 8006 Zürich (CH); Graf, Josua, 8006 Zürich (CH); Ackermann, Meret, 8006 Zürich (CH); Wolf, Martin, 8006 Zürich (CH)
(74) Representative: Braunpat AG

(57) **Abstract**

A monitoring device for monitoring oxygenation of tissue (31) comprises a near-infrared optical detection unit (100) and a probe unit (40) having a plurality of light emitters (50) directed toward the tissue (31), and at least one light detector (10) receiving light returning from the tissue (31). The probe unit (10) comprises a rigid support structure (1) that carries the plurality of light emitters (50) and/or the at least one light detector (10). The rigid support structure (1) comprises a plurality of positioning sensors (5) for detecting a positioning of the probe unit (10) relative to a tissue surface (30) located above the tissue (31) and several sensor guide elements (3) extending from the support structure (1) and moveably supporting the plurality of positioning sensors (50). The probe unit (10) further comprises a cushion structure (2) that comprises an attachment side (20) attached to the support structure (1) and a contacting side (21), opposite to the attachment side (20), having a compressible surface (22) for contacting the tissue surface (30). The plurality of positioning sensors (5) moveably extend through the cushion structure (2) towards the contacting side (21) of the cushion structure (2).

## Description

### Technical Field

The present invention relates to a monitoring device for monitoring oxygenation of human or animal tissue, particularly to a non-invasive monitoring device for time-resolved near-infrared optical tomography (TR NIROT), especially for monitoring oxygen saturation of hemoglobin, oxy- and deoxy-hemoglobin and cytochrome c oxidase concentration of cerebral or other tissues, specifically of preterm infants and young children.

### Background of the invention

Oxygenation (StO2) of body tissue is a clinically highly important physiological parameter. It reflects the balance between oxygen supply and oxygen consumption. For example, the brain is very sensitive to a lack of oxygen, which can lead to brain lesions and death. Oxygenation is also an important factor in oncology. For example, it is a marker for malignity of tumors and the effectiveness of radiation therapy depends to a large degree on oxygenation. In both instances, oxygenation varies locally and in its intensity. Therefore, it is important to be able to image and quantify oxygenation. There are many other fields were oxygenation is relevant.

Oxy- and deoxyhemoglobin (HbO₂ and HbR) concentration of body tissue are also clinically relevant parameters because hemoglobin corresponds to the main oxygen transporter in tissue and a sufficient oxygen supply is crucial for any tissue. In addition, the sum of oxy- and deoxyhemoglobin concentration corresponds to the total hemoglobin concentration, a parameter that reflects the blood flow, since the blood flow increases, when the blood vessels open up and this also leads to higher total hemoglobin concentration. Thus, the mentioned parameters enable to obtain relevant information about the body tissue.

Cytochrome c oxidase (CCO) is an enzyme at the cellular level that is oxidized and reduced depending on the oxygenation. In general, its redox state reflects the cellular oxygenation and it is therefore also highly relevant.

Near infra-red spectroscopy (NIRS) is the only non-invasive cerebral blood oxygen saturation monitoring technique available so far. NIRS-based detection of cerebral blood oxygen saturation is based on the different spectral absorption features of HbO2 and HbR in the tissues detected within the near-infrared optical window (700-950 nm). Appropriate wavelengths are chosen to estimate cerebral blood oxygen saturation from the light intensity attenuation at two or more wavelengths. Variations in the HbR and HbO2 content are calculated according to optical absorption theories.

The basic setup of NIRS-based equipment includes a light emitter or light source, a photoelectric detector, and a control unit, like a computer. The light source emits near-infrared light at certain wavelengths that penetrate tissues deeply. The photoelectric detector, for example in form of non-invasive monitoring sensors for detecting photons, measures the emergent light intensity. The control unit converts the emergent light intensity into useful clinical information. Generally, there are two types of NIRS devices, depending on the position of the photoelectric detector: (1) transmission NIRS and (2) reflectance NIRS.

NIRS-based monitoring allows for directly or indirectly detecting physiological changes and metabolic processes. The near-infrared light has a strong transmission capacity in human brain tissues. Within the optical window of 700-950 nm, the penetration depth of the near-infrared light can reach several centimeters. In comparison with existing techniques-such as computer tomography (CT), functional magnetic resonance imaging (fMRI), ultrasound, x-ray or magnetic resonance imaging, and electroencephalograms (EEG)-NIRS monitoring has a higher temporal resolution and is a non-invasive safe method for real-time continuous measurement.

NIRS monitoring has also been applied to cerebral blood oxygen saturation, HbO₂, HbR and CCO monitoring in infants and young children and is considered as the most suitable monitoring method for this age group. Studies have shown that for infants under 1 year old, there exists significant differences in cerebral blood oxygen saturation at different body positions. rSOt2 monitoring is an effective way for physicians to understand infant brain health conditions with a view to adjust the nursing procedures and protect the brain.

To address this problem two types of devices are built, standard NIRS monitoring devices have no spatial resolution, i.e. the measure at one location and continuously measure oxygenation. This type of device is widely used, but since it has no spatial resolution, it is often assumed that the measured volume of tissue represents a whole organ. This assumption is wrong in many cases, for example it is known that brain lesions in preterm infants occur in specific locations, which implies that oxygenation is not the same between different volumes of the brain. The same is true in tumors, which are known to have highly variable oxygenation between different parts of the tumor. For this reason, the next step is to build NIRS devices which enable a spatial resolution, i.e. with imaging capabilities. These devices are called near-infrared optical tomographs or diffuse optical tomographs.

One example of an oxygenation monitoring system based on NIRS uses high-density diffuse optical tomography (HEATING DRUM-DOT) as described in US 2017/0231501 A1. The system includes a wearable head apparatus and an electronic console. The head apparatus receives resultant light from the head of a subject. The electronic console includes a fiber array, a detector, and a computing device. The fiber array includes a plurality of fibers for transporting resultant light received by the head apparatus. The detector includes a plurality of photo detectors each associated with a fiber. Each photo detector generates a plurality of detection signals in response to detected resultant light from its associated fiber. The computing device receives the detection signals from each of the plurality of photo detectors and generates a high density-diffuse optical tomography (HD-DOT) image signal of the brain activity. Another example of a NIRS device and method is shown in EP 4023144 A1. The NIRS device comprises a data processing unit, a plurality of NIR emitters for emitting light and a plurality of NIR detectors for receiving light. The emitters and detectors are coupled with a head mount, like a cap or a patch, to be worn by a person on the head. The emitters and detectors are arranged in a pattern on the head mount, such that the head mount and the pattern define the positioning of the detectors, when monitoring tissue oxygenation.

A further example of a NIRS device is the time-resolved near-infra-red optical tomography (NIROT) system 'Pioneer', which is developed for imaging oxygenation of the preterm brain. In preterm infants, there is a risk of long-term cognitive, motor and behavioral impairments due to hemorrhagic and/or ischemic lesions. If detected early, lesions can be prevented. Pioneer employs multiple wavelength laser light in short pulses on eleven distinct locations and measures the reemerging light in a contactless fashion by means of a time-correlated single-photon counting camera covering ~4.9 cm² with 300 detectors. This instrument measures the time-of-flight of the photons, i.e. how long the photons need to travel through the tissue. This so-called time-domain approach provides a further dimension of information that enables to determine the optical properties of tissue and reflects how deep photons have travelled into the tissue. A biocompatible ring structure allows for fixed relative positions of sources and detectors and provides a secure interface between the patient and the probe.

However, NIRS monitoring also has some drawbacks. For example, its spatial resolution are low, and selection of the monitoring site has a large influence on the results. Particularly for NIRS imaging devices these technical inefficiencies lead to poor results. Different commercial monitoring equipment tend to use different algorithms; therefore, it is difficult to compare the monitoring results across equipment, and no standards have yet been established. Further, rSO2 presents extensive intra- and interindividual baseline variability. No definite normal range of rSO2 has been established and there is no common standard to determine the thresholds for recognizing cerebral ischemia or hypoxia.

For NIROT/DOT imaging devices mostly, the positioning of the probe, particularly of the detectors and detector arrays, towards the monitored tissue, i.e. the distance between the detectors and the tissue, is vague and unprecise and varies from person to person, since the surface of the tissue (e.g. the head) varies. This affects quantitatively accurate detection of oxygenation, HbO2, HbR and CCO of clearly defined brain tissue areas and therefore hinders adequate surveillance and treatment of the person. In summary, NIRS-based rSO2 monitoring equipment is generally only used for trend analysis.

It is an object of the present invention to provide a device for monitoring oxygenation, HbO2, HbR and CCO of cerebral and other tissue based on near-infrared optical detection that provides accurate imaging and quantification of tissue oxygenation and precise positioning determination of the monitoring device on a person's head, that allows for simple handling and does not affect the monitored person, and that can be assembled, cleaned and maintained in an easy manner. Particularly, it is an object of the present invention to provide an oxygenation monitoring device that allows for precise detection of the cerebral tissue oxygenation of preterm infants and very young children without affecting their health and well-being.

### Summary of the invention

The above-mentioned objects and other objects are fulfilled by a monitoring device for monitoring oxygenation of human or animal tissue and a probe unit for such a monitoring device according to independent claims 1, 15 and 16. Advantageous features and preferred embodiments are disclosed in dependent claims.

A monitoring device for monitoring oxygenation, particularly for monitoring oxy- and deoxyhemoglobin concentration, oxidized and reduced cytochrome C oxidase, and other potential physiological parameters of a human or animal tissue as provided by the present invention comprises a near-infrared optical detection unit having a plurality of light emitters directed toward the tissue and at least one light detector receiving light returning from the tissue. For example, the light emitters can be connected to optical fibers providing the NIR light. Also, as common for NIROT devices, the light emitters and light detectors can be interchangeable, i.e. light emitters can be used as light detectors and vice versa. The monitoring device for monitoring oxygenation is particularly suitable for monitoring physiological parameters of a cerebral tissue.

According to the invention, the monitoring device comprises a probe unit having a rigid support structure and a cushion structure. The rigid support structure carries the plurality of light emitters and/or the at least one light detector, or is connected to the at least one light detector. The support structure comprises a plurality of positioning sensors for detecting a positioning of the probe unit relative to a surface of the tissue or a surface close by the tissue, like a surface located immediately adjacent to the tissue, for example a head surface close by a cerebral tissue. Further, the support structure comprises several sensor guide elements extending from the support structure and moveably supporting the plurality of positioning sensors. For example, the sensor guide elements extend from the support structure in a direction away from the tissue, e. g. perpendicular from a carrier base of the support structure. The cushion structure comprises an attachment side attached to the support structure and a contacting side, opposite to the attachment side. At the contacting side the cushion structure has a compressible surface for contacting the tissue surface. The support structure and cushion structure together build a basis for the probe unit of the monitoring device that is set for example on a person's head surface for monitoring the oxygenation of cerebral tissue. For example, the probe unit is put on the head surface above the cerebral tissue area that is to be monitored. Due to its compressible nature the surface adjusts to a shape of the head surface resulting in areas of the cushion structure of higher deformation and other areas with less or no deformation. According to the invention the plurality of positioning sensors moveably extend through the cushion structure towards the contacting side of the cushion structure. Therefore, when the probe unit is put on the tissue surface and the compressible surface of the cushion structure is deformed, the plurality of positioning sensors move from an initial position to a partially shifted or deformed position that corresponds to the characteristic surface shape of the tissue surface, e.g. the head surface, of the person. Advantageously, x- and y-coordinates of the plurality of positioning sensors are known from their mounting position at the probe unit. The z-coordinate can be determined by each of the positioning sensors. Thus, a set of 3D coordinates can be acquired and used for generation of the tissue surface monitored by the monitoring device.

The plurality of positioning sensors captures the three-dimensional form of the tissue surface, which contributes to exact localization and quantification of oxygenation measures captured by the near-infrared optical detection unit. Taking the individual three-dimensional form of the tissue, respectively a surface of the tissue, into account allows for accurate and reproducible determination of the oxygen saturation for varying individuals. At the same time, it is easy to separate the flexible cushion structure from the positioning sensors for cleaning of the compressible surface and maintenance of the probe unit.

The near-infrared optical detection unit may be designed as known from the state of the art, for example as developed for the time-resolved near-infra-red optical tomography (NIROT) system 'Pioneer'. Particularly, the near-infrared optical detection unit may comprise a laser unit providing near-infrared light for the light emitters, a camera system or optode as light detector and an optical power sensing system. Further, the near-infrared optical detection unit may comprise or be connected to a control unit for controlling light emission of the plurality of light emitters and a processing unit processing information data captured by the at least one light detector. The control unit and/or processing unit can be used to control the positioning sensors and process the data sets captured by the positioning sensors, respectively. The light emitters fed by the optical fibers can be hosted inside the cushion structure, with each light emitter being embedded in a transparent window in the compressible surface. Also, the light emitters could be connected to the positioning sensor to be guided to the tissue surface.

The monitoring device of the invention provides an accurate image reconstruction derived from the precise information about the tissue surface shapes and locations of light emitters and detectors, e. g. pixels of a camera. The probe unit provides a flexible interface in form of the cushion structure with the ability to determine the shape of the tissue surface, for example the head surface above a cerebral tissue. The flexible cushion structure provides the interface between the probe unit and the tissue or object to be investigated. It adapts to the surface of the head and the tissue, respectively, to achieve tight contact, not exert undue pressure on the tissue and report the precise location of all the light emitters and light detectors.

In one embodiment of the monitoring device according to the present invention the support structure carries a positioning sensor at least for each light emitter. Thus, there is at least the same number of positioning sensor as there are light emitters. Each light emitter has an assigned positioning sensor, that captures a position of that light emitter and in relation to other light emitters, the at least one detector and the probe unit, respectively. The tissue can be divided into several subareas, wherein each tissue subarea is irradiated by one or more specific light emitters. Knowing the positioning of said specific light emitters towards the surface of the tissue partition allows for accurate evaluation of oxygenation in said tissue subarea. The results of the several subareas together provide a holistic result of the oxygenation of the tissue. Advantageously, the support structure may carry additional position sensors, which are not assigned to a light emitter for further detecting the surface shape of the tissue of interest. Thus, the number of position sensors can be higher than the number of light detectors. Vice versa, the number of position sensors can also be lower, e.g. when the shape of the tissue is uncomplicated.

Preferably, each of the plurality of positioning sensors moves independently at the sensor guide element said positioning sensor is attached to. Advantageously, the positioning sensors comprise a sensor body and a sensor shaft carrying a sensor tip and being slidable supported in the sensor body. For example, the positioning sensors are designed as potentiometer sensors having an extendable and retractable shaft carrying a sensor tip for sensing a surface. The sliding axis of the sensor shaft defines the z-direction as mentioned above for the three-dimensional data set defining the positioning of the positioning sensor, light emitters and light detectors. However, any other suitable types of sensors to determine the position may be used.

Preferably, sensor tips of the plurality of positioning sensors extend through the cushion structure to flush with the compressible surface. That means the sensor tips of the positioning sensors are leveled with the plane of the compressible surface. However, the tips do not necessarily need to contact the compressible surface, rather the are located next to the compressible surface. Before setting the probe unit on a person's head, the positioning sensors can be described as being in an initial or zero state with their sensor tips extended. When the probe unit is positioned on a tissue surface, at least some areas of the compressible surface are deformed, which causes the positioning sensors, respectively the sensor tips, at or next to the compressible surface to at least partially move, which can be described as a monitoring or moved state of the positioning sensors. This arrangement allows for precise location detection without exerting uncomfortable forces onto the tissue surface.

In one embodiment of the monitoring device according to the invention, the sensor guide elements extend from a first side of the support structure and the cushion structure is attached to a second side of the support structure opposite to the first side. For example, the support structure can have a ring shape or disc shape comprising a central opening for allowing light re-emerging from the tissue to reach the at least one detector. Preferably, the sensor guide elements are arranged on the first side of the support structure such to provide a circle of positioning sensors around the central opening. Advantageously, there is an outer circle of positioning sensors and additionally an inner circle of positioning sensors. For example, the plurality of sensor guide elements are realized as rod-shaped elements, and advantageously extend perpendicular from the first side of the support structure. They can guide the positioning sensors by defining a moving or sliding direction for the movement of the sensor tips. In general, the design and orientation of the sensor guide elements of the support structure depends on the type of positioning sensor used. While perpendicular sensor guide elements are useful to support potentiometer sensors as described above, other sensor designs may require a different set up of the sensor guide elements.

The cushion structure may for example be made of biocompatible flexible material, like foam or silicone material, which can be formed to a desired shape for example by using a mold cast. Thus, the cushion structure can be designed as an entirely flexible, one-piece unit, that builds the interface between the head of a person and the support structure connected to the at least one detector and the near-infrared optical detection unit. At the attachment side, the shape of the cushion structure may be designed such that it engages in a form fit with the support structure. The cushion structure can simply be attached to the support structure by stretching the flexible material and forcing it onto the support structure, where it engages in a tight fit with the support structure. For example, the cushion structure may comprise an outer rim with an inwardly extending protrusion that can be stretched and forced onto an outer edge of the support structure. Of course other types of attachment means may be used, which preferably are designed to releasably attach the cushion structure with the support structure.

Advantageously, the compressible surface of the cushion structure may form a circle along an outer rim of the contacting side of the cushion structure and an inner free space is realized within such a compressible surface circle. The positioning sensors can reach into the inner free space of the cushion structure. Thus, the structure does not interfere with any movement and measurements of the positioning sensor.

For monitoring the oxygenation, the HbO2, HbR and CCO level, and other potential physiological parameters and the distribution of the tissue, the probe unit of the monitoring device is put on the tissue surface or a surface next to the tissue as mentioned above and NIR light can be radiated into the tissue. Light returning from the tissue can pass through the inner free space of the cushion structure and through the central opening of the support structure to the at least one light detector without disturbances. The position of the at least one light detector relative to the monitored tissue can be determined using the three-dimensional data set provided by the positioning sensors, which accurately indicate the orientation and distance of the at least one light detector with respect to the surface and the tissue, respectively. Consequently, precise monitoring of the oxygenation of the tissue is possible.

In a further embodiment of the monitoring device according to the present invention an optical fiber carrying a light emitter is attached to a positioning sensor. Thus, the position of the light emitter relative to the tissue surface is precisely detected by the moveable positioning sensor. Advantageously, a light emitting end of the optical fiber aligns with the tip of a positioning sensor. The position of the light emitter may correspond to the position of the sensor tip. The optical fibers are able to move relative to the support structure together with positioning sensors. Consequently, any interaction of the emitted NIR light with the tissue can be closely monitored and precise monitoring of the oxygenation of the tissue is possible.

In a still further embodiment of the monitoring device according to the present invention the cushion structure comprises a plurality of flexible panel elements extending with one end from the contacting side of the cushion structure towards the tissue surface and carrying contact faces at their other ends for contacting the tissue surface. The contacting faces provide the compressible surface because the flexible panel elements are compressed and bend when they are pushed against the tissue surface. By choosing the design of the flexible panel elements the compressibility of the cushion structure can be adjusted to the needs of the monitoring device. For example, the flexibility can be determined by choosing shape, thickness and length of the flexible panel elements. The cushion structure for example comprises a ring-shaped or disc-shaped base, preferably corresponding to the shape of the support structure. The flexible panel elements are arranged with one end at the contacting side of the base and extend therefrom perpendicular or in an angle. The other end of the flexible panel elements comprises the contacting faces. Several flexible panel elements can carry the same contacting face. Also, the contacting faces maybe connected. Preferably, the contact faces run parallel to the base of the cushion structure and therefore parallel to a carrier base of the support structure, when the cushion structure is mounted to the support structure.

In an advantageous variant, the plurality of flexible panel elements in a relaxed state have a curved shape. In a compressed state, for example when pushed on the tissue surface, they have an even more curved shape. The curved shape predefines a bending direction for the flexible panel elements. The curved shape of the flexible panel elements facilitates the compression of the compressible surface and the plurality of contacting faces. Therefore, less pressure is required to adapt the compressible surface and with it the position of the positioning sensor when monitoring the oxygenation of the tissue.

In a preferred embodiment of the monitoring device the plurality of flexible panel elements of the cushion structure are designed such that a pair of flexible panel elements form an O-shape, wherein one side of the O-shape is oriented towards the attachment side and an opposing side of the O-shape carries contacting faces. In an initial relaxed state of the flexible panel elements, the O-shape is elongated and the contacting faces are most distant from the cushion structure base. In a compressed state the O-shape is flattened and the contacting faces are less distant from the cushion structure base. The O-shape of the flexible panel elements allows for independent compression of different areas of the compressible surface and the contacting faces, respectively. As mentioned earlier, the compressible surface advantageously forms a circle along an outer rim of the contacting side of the cushion structure and an inner free space is realized within the compressible surface circle. In this variant the flexible panel elements and the contacting faces can be arranged in a circle along the outer rim of the contacting side of the cushion structure. This allows for an extended free space and facilitates precise measurements of the returning light by the at least one light detector.

In an alternative embodiment of the monitoring device the plurality of flexible panel elements of the cushion structure are aligned in a zigzag pattern between the cushion structure base and the compressible surface. For example, upper tips of the zigzag pattern are attached to the cushion structure base and opposing lower tips of the zigzag pattern carry the contact faces or a circular compressible surface. Again, individual areas of the compressible surface can be deformed by pressing against the flexible panel element, which will be deformed by the pressure force.

In all these embodiments, optical fibers can also be employed for the detection of light or photoelectric detectors can be implemented directly in the probe and therefore the positioning sensors can also be attached to the detectors or detection fibers.

For calibrating the monitoring device according to the present invention an innovative two-step calibration method was used, adapted to the specific features of the monitoring device. In a first calibration step a linear function for determining surface measures is generated using the positioning sensor on a set of calibration structures. In a second calibration step the linear function is verified using the positioning sensor on a set of shape samples, for example a tissue shape sample or surface shape sample, particularly a set of head shape samples, and the linear function can be fine-tuned if necessary.

In an advantageous variant of the calibration method according to the present invention, in the first calibration step the support structure is used for the calibration measurements with the plurality of light emitters, the at least one light detector and the plurality of positioning sensors connected thereto but without an attached cushion structure. For the second calibration step, the same support with the plurality of light emitters, the at least one light detector and the plurality of positioning sensors connected thereto is used with an attached cushion structure. Further details of the calibration method according to the present invention are explained below.

The monitoring device for monitoring oxygenation of tissue according to the present invention is able to directly detect not only physiological changes but also quantified levels of oxygen saturation. The oxygenation measurements are precise and reproducible because the positioning of the monitoring device is clearly defined. Moreover, the penetration depth of the near-infrared light can be monitored closely, and the monitoring provides a higher temporal resolution for real-time continuous measurement.

### Brief description of the drawings

Several exemplary embodiments of the monitoring device according to the invention will be illustrated in the following drawings, which merely serve for explanation and should not be construed as being restrictive. The features of the invention becoming obvious from the drawings should be considered to be part of the disclosure of the invention both on their own and in any combination. The drawings show:
- Fig. 1a:: a three-dimensional top view of an embodiment of a support structure of a monitoring device according to the invention,
- Fig. 1b:: a three-dimensional bottom view of the support structure of figure 1a,
- Fig. 2a:: a side view of a first embodiment of a cushion structure of a monitoring device according to the invention,
- Fig. 2b:: a three-dimensional bottom view of the cushion structure of figure 2a,
- Fig. 3a:: a three-dimensional top view of a second embodiment of a cushion structure of a monitoring device according to the invention,
- Fig. 3b:: a three-dimensional bottom view of the second embodiment of figure 3a,
- Fig. 4a:: a three-dimensional view of an assembled support structure and cushion structure,
- Fig. 4b:: the three-dimensional view of the assembled support structure and cushion structure of figure 4a attached to a light detector,
- Fig. 5:: a schematical diagram of a positioning sensor of a monitoring device according to the present invention, and
- Fig. 6:: a schematical diagram of a monitoring device according to the present invention.

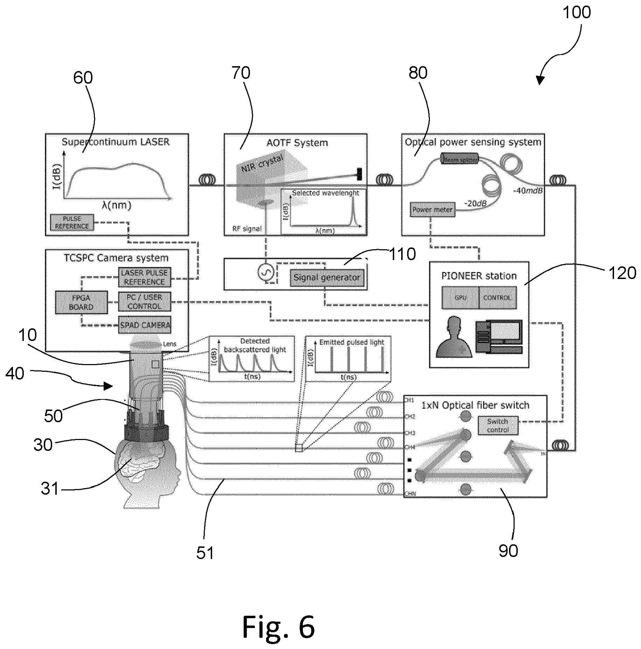

The monitoring device of the invention is described in the figures in more detail by explaining its application for monitoring the oxygenation of a cerebral tissue 31 located below a head tissue surface 30 as schematically illustrated in figure 6. However, the tissue 31 could be another type of tissue and the tissue surface 30 is defined by the outer surface of the body just next to the tissue 31.

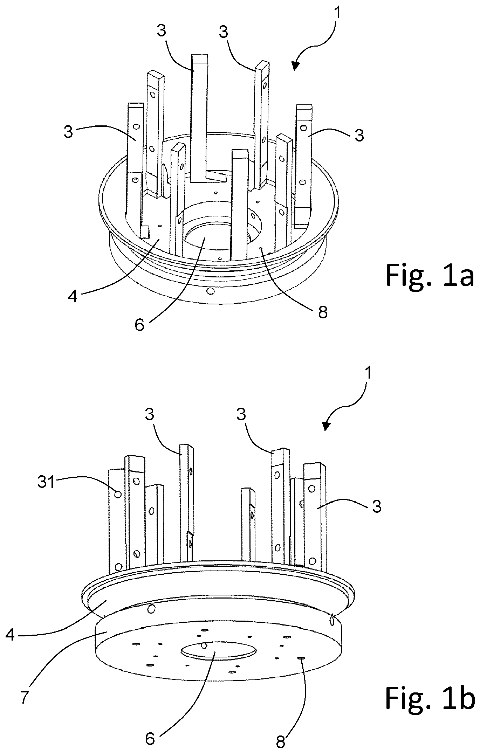

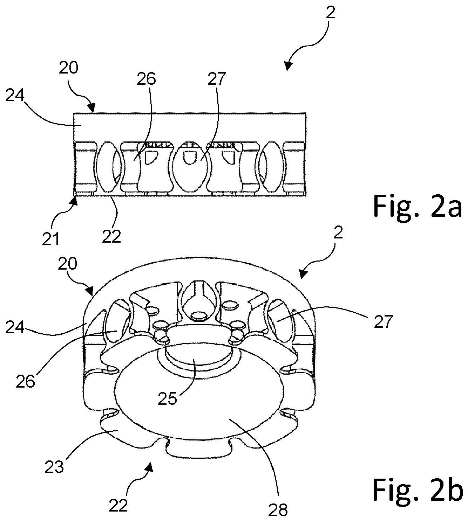

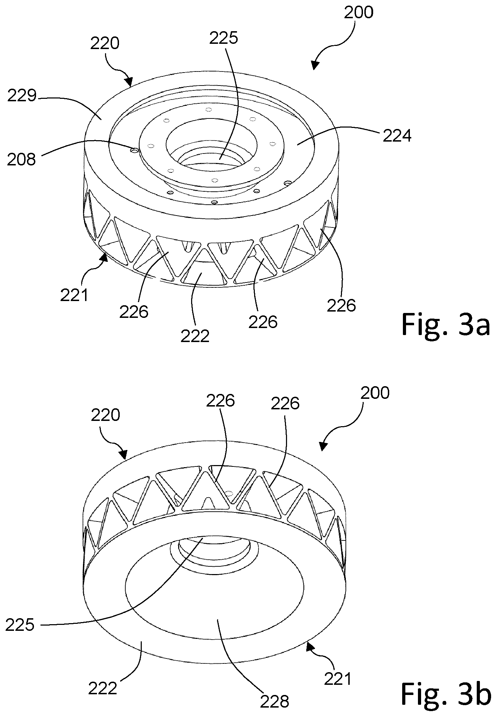

Figures 1a and 1b show detailed views of an embodiment of a rigid support structure 1, figures 2a and 2b show detailed views of a first embodiment of a cushion structure 2 and figures 3a and 3b show detailed views of a second embodiment of a cushion structure 200 for a probe unit 40 of a monitoring device (see figure 6) for monitoring oxygenation of tissue according to the present invention.

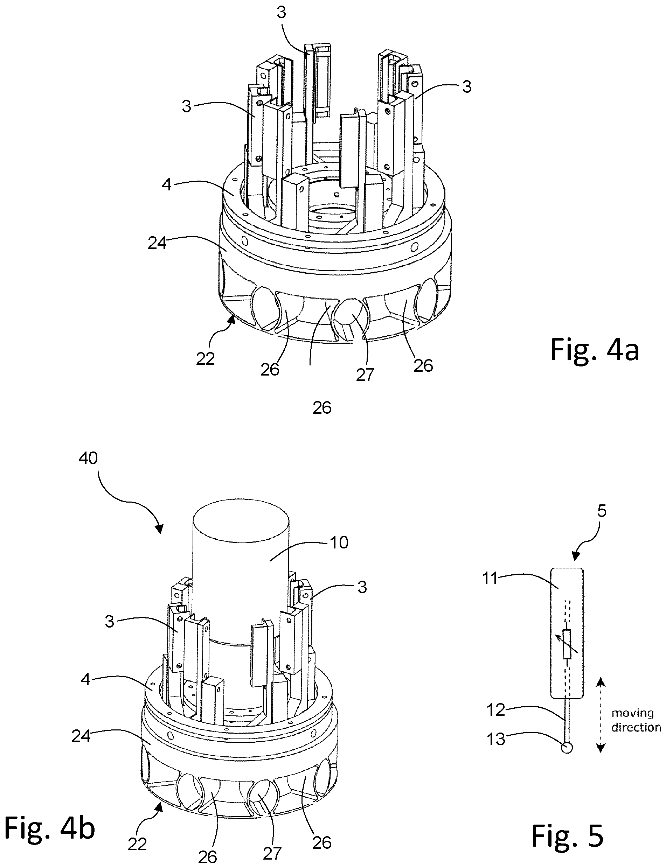

Figure 1a illustrates a top side of the rigid support structure 1, which is designed to carry a plurality of light emitters, at least one light detector and a plurality of positioning sensors 5 for detecting a positioning of the probe unit 40 relative to a head surface 30 located next to an area of cerebral tissue 31 (see figure 6). The support structure 1 comprises several sensor guide elements 3 extending from a ring-shaped carrier base 4 on the top side of the support structure 1. The sensor guide elements 3 extend perpendicular from the carrier base 4. The sensor guide elements 3 are designed to support the moveable positioning sensors 5 (see figure 5). Each sensor guide element 3 comprises mounting means like holes for attaching a sensor body 11 of the positioning sensors 5 to the sensor guide element 3. The support structure 1 comprises a central opening 6 of 3-7 cm², for example. The sensor guide elements 3 are arranged in a circle around the central opening 6. As can be seen in figure 1b showing a bottom side of the support structure 1, on the bottom side the carrier base 4 comprises a radially extending circular attachment rim 7 for fastening the cushion structure 2 or 200 to the support structure 1. The support structure 1 comprises a plurality of through holes 8, which allow the positioning sensors 5 to extend from the top side to the bottom side of the support structure 1. The support structure 1 is made of plastics or metal, for example.

Figure 2a shows the first embodiment of the cushion structure 2 from the side and figure 2b shows a bottom view. The cushion structure 2 is made entirely of flexible material, like silicone. The cushion structure 2 comprises an attachment side 20, upper side in figures 2a and 2b, to be attached to the support structure 1, and a contacting side 21, opposite to the attachment side 20, for contacting the tissue surface. The contacting side 21 has a compressible surface 22 for contacting the tissue surface. As illustrated in figure 2b, the compressible surface 22 is realized by several contact faces 23. The cushion structure 2 is ring-shaped and comprises a base ring 24 with a central opening 25. The base ring 24 is designed as attachment means for attachment to the rim 7 of the support structure 1. An example of the attachment means is described for the second embodiment of the cushion structure 200. Due to the flexible nature of the material, the base ring 24 can be force fitted onto the rim 7 of the support structure 1. A plurality of flexible panel elements 26 extend from the base ring 24 towards the compressible surface. The panel elements 26 are arranged in a circle around an outer circumferential area of the base ring 24. Inside that circle the panel elements 26 and the base ring 24 define an inner free space 28 that is open towards the contacting side of the cushion structure 2. In the first embodiment of the cushion structure the panel elements 26 have a curved design and a pair of flexible panel elements 26 form an O-shape 27. One end of the O-shape starts at the attachment side and an opposing end of the O-shape carries a contacting face 23. The contact faces 23 are linked with each other at one of their corners. The contact faces 23 define a ring-shaped compressible surface 22. Alternatively, the contacting faces could be linked at several linking spots. Also, the contacting faces 23 could be realized by a single continuous ring-shaped circular face that serves as the compressible surface 22.

Figure 3a shows the second embodiment of the cushion structure 200 from a top side and figure 3b shows a bottom side. The cushion structure 200 is also made entirely of flexible material, like silicone. The cushion structure 200 is ring-shaped and comprises a base ring 224 with a central opening 225. The cushion structure 200 comprises an attachment side 220 and a contacting side 221, opposite to the attachment side 220. At the attachment side 220, there is a lip ring 229 extending slightly upwards and then inwards from an outer circumference of the base ring 224. The lip ring 229 is elastic and can be forced onto the attachment rim 7 of the support structure 1 for releasably fastening the cushion structure 200 to the support structure 1. The contacting side 221 has a continuous ring-shaped compressible surface 222 for contacting the tissue surface. The compressible surface 222 serves as a contacting face that can be put on the tissue surface. A plurality of flexible panel elements 226 extend from the base ring 24 towards the contacting side 221. The panel elements 226 are arranged around the outer edge of the base ring 24. In the second embodiment of the cushion structure the panel elements 226 are straight panels that extend in an angle from the base ring 224. One end of the panel elements 226 starts at the base ring 224 and an opposing end of the panel elements 226 terminates at the compressible surface 222. The panel elements 226 extend in a zigzag pattern between the base ring 224 and the compressible surface 222 around the central opening 225 at an outer circumference of the cushion structure 200. The panel elements 226 and the base ring 224 define an inner free space 228 that is open towards the contacting side of the cushion structure 200. The base ring 224 comprises several through holes 208, which correspond to the through holes 8 of the support structure 1 and allow the positioning sensors 5 to extend towards the contacting side 221 of the cushion structure 200.

Figure 4a illustrates the support structure 1 and the cushion structure 2 attached thereto, together building the base structure for the probe unit 40. As can be seen the outer circumference of the cushion structure 2 matches the outer circumference of the support structure 1. The carrier base 4 of the support structure 1 and the base ring 24 of the cushion structure 2 are facing each other. The several sensor guide elements 3 extend upwards, away from the cushion structure 1 and the panel elements 26 extend downwards in direction of the compressible surface 22 away from the support structure 1. The central opening 6 of the support structure 1 aligns with the central opening 25 of the cushion structure 2. The inner free space 28 of the cushion structure 1 amidst the panel elements 26 is open towards the compressible surface 22. The through holes 8 align with the through holes in the base ring 24 of the cushion structure 2. In figure 4b the probe unit 40 is attached to a light detector 10 in form of a detector tube such that the light detector is above the central opening 6. In this embodiment the light detector comprises a tube structure to collect light and is connected to a camera. In that position the light detector 10 can detect any backscattered reemerging light from the tissue that enters the probe unit 40 through the inner free space 28 of the cushion structure 2 in a contactless fashion.

Figure 5 schematically illustrates an example of a positioning sensor 5 in form of a potentiometer. The sensor comprises a fixed sensor body 11 and a sensor shaft 12 with a sensor tip 13. The sensor shaft 12 is slidably arranged in the sensor body 12, which allows for moving the sensor tip 13 unidirectional for sensing a position. The positioning sensors 5 are attached to the sensor guide elements 3, wherein the sensor bodies 11 are mounted to the sensor guide element 3 and the sensor shafts 12 extend through the support structure 1 and the cushion structure 2 in direction of the compressible surface 22. For example, the sensor shafts 12 extend through the through holes of the support structure and the cushion structure. In one example, the sensor shafts 12 can be surrounded by a compressible sheath, which supports the shaft and guides the movement of the shaft. The compressible sheath can be realized by two end rings connected by a number of flexible bars, together building a tube-like structure. When compressing the sheath the bars extend outwards against their tension force, which for example prevents the sensor shaft 12 from escaping sideways.

The positioning sensors 5 have a one-dimensional free movement in the z-axis. A readout from the positioning sensors 5 can be used for determination of the surface shape of a surface or a surface close to the tissue, as mentioned earlier. The sensor tips 13 touch the head surface once the probe unit 40 is placed on the head surface and are moved according to the head surface shape. From the readout of the displacement of the sensor shaft 12 in the z-axis from several positioning sensors 5, a surface shape of the head can be reconstructed. A number of the position sensors 5 are connected to optical fibers 51 to obtain precise locations of the light emitters 50.

The movement of the sensor tips 13 serves as an indicator for the orientation and distance of the probe unit 40 relative to the tissue surface. The positioning sensors 5 are adjusted such that in a relaxed initial state of the compressible surface 22 the sensor tips 13 are calibrated to a zero position, wherein the sensor tips are essentially leveled with the compressible surface. Thus, the sensor tips 13 of the plurality of positioning sensors 5 extend through the cushion structure 2 to flush with the compressible surface 22. When the probe unit 40 is put onto a tissue surface 30 with slight force, the compressible surface 22 is compressed by the surface shape of the tissue surface and the sensor tips 13 are moved towards the sensor bodies 11, wherein the sensor shafts 12 are slid into the sensor bodies 11. The displacement of the sensor shafts 12 initiates a positioning measurement for each positioning sensor 5.

Figure 6 shows the monitoring device and describes a method for monitoring oxygenation of cerebral tissue according to the present invention. The monitoring device comprises a near-infrared optical detection unit 100 having a plurality of light emitters 50 connected to optical fibers 51 and the light detector 10 in form of a camera system comprising for example a time-correlated single-photon counting (TCSPC) camera chip. It comprises an array of 32x32 single-photon avalanche diodes and a temporal resolution of 116 ps. This high density of detectors provides a large amount of time-resolved data for image reconstruction. The light emitters 50 at the end of the optical fibers 51 are directed toward tissue 31. In this embodiment the optical fibers 51 can be hosted inside the cushion structure 2 or 200, with each fiber tip as the light emitter 50 being embedded in a transparent silicone window. Alternatively, the optical fibers 51 and the light emitters 50, respectively, can be supported by the sensor guide elements 3. Advantageously, each light emitter 50 is associated to one positioning sensor 5. The position of the light emitters 50 and the position of the light detector 10 at the probe unit 40 are well defined on the x- and y-axis. The position in z-axis is determined by the positioning sensors 5. Thus, the exact distance to and from the tissue and areas of light deflection can be precisely determined. The near-infrared optical detection unit 100 further comprises a laser 60, a near infra-red device 70 with a NIR crystal, an optical power sensing system 80, an optical fiber switch unit 90, a signal generator 110 and a processing/control unit 120.

The function of the near-infrared optical detection unit 100 is commonly known. For example, the unit 100 operates like the time-resolved near-infra-red optical tomography (NIROT) system 'Pioneer', which is configured to measure three-dimensional tissue oxygenation. The laser 60 is realized as a supercontinuum laser which generates pulsed light in a broadband wavelengths range. The laser light is coupled to the near infra-red device 70 in form of an acousto-optical tunable filter (AOTF), which selects a specific laser wavelength of 4 nm bandwidth. The AOTF wavelength selection-range ranges for example from 650 nm to 900 nm. The optical power sensing system 80 may comprise a fiber-splitter with a tap-channel power meter, which monitors the light intensity. Thus, the output light power can be included in the reconstruction process. The optical fiber switch 90 comprises for example one input and 24 output channels, and allows for switching the light emission between several of the optical emitters 50. The optical fibers 51 are for example realized as graded-index optical fibers and guide the light to the tissue 31. The light is emitted from the light emitters 50 with a few mW optical power. It penetrates the skin and interacts with the tissue. The processing/control unit 120 receives an information signal about the stimulating input light of the light emitters 50 from the signal generator 110, a detection signal from the camera system 60 and positioning signals from the positioning sensors 5. The processing/control unit 120 for example comprises an embedded reconstruction algorithms configured to determine the positioning of the light emitters 50 and/or the light detector 10 relative to the tissue 31 and to produce a three-dimensional reconstruction of the tissue oxygenation.

For calibrating the monitoring device according to the present invention, the innovative two-step calibration method is used as mentioned above. In a first calibration step a linear function for determining surface measures is generated using the positioning sensor on a set of calibration structures. The calibration structures can for example be produced by a three-dimensional printing process. For example, the two calibration structures are realized as (1) a large two-step flat surface structure with a first step of 8mm for an outer circle of positioning sensor and a second step of 20mm for an inner circle of position sensors, and (2) a little two-step flat surface structure with a first step of 4mm for an outer circle of positioning sensor and a second step of 11mm for an inner circle of position sensors, wherein the step height is measured relative to a reference plane corresponding to an initial or zero displacement position of the positioning sensor. A series of at least two surface shape test measurements was performed using the support structure with the plurality of light emitters, the at least one light detector and the plurality of positioning sensors connected thereto but without attaching the cushion structure. Thus, the positioning sensors are not influenced by the presents of the cushion structure which eliminates the cushion structure as a source of measurement error. The optical fibers are fixed to the sensor shafts and the sensor tips of the positioning sensors, in order to deliver the light to the surface to be measured and at the same time obtain precise locations of the light sources.

Since the position sensor readout is linear to the movement of the sensor tips of the positioning sensors, two displacements were measured for each positioning sensor. From the two measurements, two sets of voltage values were read out. Knowing the actual displacement values for each sensor given by the known dimensions of the calibration structure a linear function of the position value with respect to the voltage readout was generated for each of the linear positioning sensors.

In a second calibration step the linear function is verified using the plurality of positioning sensors on a set of defined shape samples, for example a set of tissue shape samples or surface shape samples. Preferably, for the second step the same support structure with the plurality of light emitters, the at least one light detector and the plurality of positioning sensors connected thereto and with an attached cushion structure is used. The set of shape samples may for example be realized by two different head mimicking surface shapes: (1) semi-spherical head shape samples with different sizes, each having a radius of for example 5 cm, 6 cm, and 8 cm, and (2) semi-ellipsoidal head shape samples, each having a lengths of the semi-axes of 52 mm,48 mm and 47 mm. The shape samples can for example be produced by a three-dimensional printing process.

The shape samples are measured with the probe unit as described previously, i.e. with the cushion structure attached to the support structure. After obtaining the values from the positioning sensors, the surface shapes and the positions of sources and detectors were reconstructed using a reconstruction algorithm based on the linear function developed from the measurements in the first calibration step. The reconstructed surface shape can be displayed as a three-dimensional image and compared with the defined dimensions of the shape samples. Test calibration measures showed that a top surfaces of interest (SoV) of the reconstructed surface shape show a high agreement with the reference shapes of the shape samples. Adjustments of the linear function developed in the first calibration step were not necessary.

Based on the positioning information provided by the positioning sensors of the monitoring device according to the present invention the three-dimensional reconstruction of the tissue oxygenation is accurate and precise quantification of oxygen saturation is possible. The monitoring device allows for processing large datasets in a compact design and will lead to high quality images. The monitoring device is designed to be applicable in intensive care of extremely preterm neonates where it is particularly critical to measure the brain oxygenation during the first 72 hours of life.

### Reference Numbers

- 1: support structure
- 2: cushion structure
- 3: sensor guide element
- 4: carrier base
- 5: positioning sensor
- 6: central opening support structure
- 7: attachment rim
- 8: through holes
- 10: light detector
- 11: sensor body
- 12: sensor shaft
- 13: sensor tip

- 20: attachment side
- 21: contacting side
- 22: compressible surface
- 23: contact face
- 24: base ring
- 25: central opening cushion structure
- 26: panel element
- 27: O-shape
- 28: inner free space
- 30: tissue surface
- 31: tissue
- 40: probe unit
- 50: light emitter
- 51: optical fiber
- 60: laser
- 70: near infra-red device
- 80: optical power sensing system
- 90: optical fiber switch unit
- 100: near-infrared optical detection unit
- 110: signal generator
- 120: processing/control unit
- 200: cushion structure
- 208: through holes
- 220: attachment side
- 221: contacting side
- 222: compressible surface
- 224: base ring
- 225: central opening cushion structure
- 226: panel element
- 228: inner free space
- 229: lip ring

## Claims

1. Monitoring device for monitoring oxygenation, particularly for monitoring oxy- and deoxyhemoglobin concentration, oxidized and reduced cytochrome C oxidase, of a tissue (31) comprising a near-infrared optical detection unit (100) and a probe unit (40) having a plurality of light emitters (50) ) directed toward the tissue (31), and at least one light detector (10) receiving light returning from the tissue (31),
**characterized in that**
the probe unit (40) comprises:
- a rigid support structure (1) that carries the plurality of light emitters (50) and/or the at least one light detector (10), and comprises a plurality of positioning sensors (5) for detecting a positioning of the probe unit (40) relative to a tissue surface (30) located above the tissue (31) and several sensor guide elements (3) extending from the support structure (1) and moveably supporting the plurality of positioning sensors (5), and
- a cushion structure (2) that comprises an attachment side (20) attached to the support structure (1) and a contacting side (21), opposite to the attachment side (20), having a compressible surface (22) for contacting the tissue surface (30),
wherein the plurality of positioning sensors (5) moveably extend through the cushion structure (2) towards the contacting side (21) of the cushion structure (2).

2. Monitoring device according to claim 1, wherein the support structure (2) carries a positioning sensor (5) at least for each light emitter (50).

3. Monitoring device according to one of the preceding claims, wherein sensor tips (13) of the plurality of positioning sensors (5) extend through the cushion structure (2) to flush with the compressible surface (22).

4. Monitoring device according to one of the preceding claims, wherein the support structure (1) comprises at least an outer circle of positioning sensors (5) and an inner circle of positioning sensors (5).

5. Monitoring device according to one of the preceding claims, wherein the compressible surface (22) of the cushion structure (2) forms a circle along an outer rim of the contacting side (21) of the cushion structure (2) and an inner free space (28) is realized within the compressible surface circle.

6. Monitoring device according to one of the preceding claims, wherein each of the plurality of positioning sensors (5) move independently at the sensor guide element (3) a positioning sensor (5) is attached to.

7. Monitoring device according to one of the preceding claims, wherein each of the plurality of positioning sensors (5) move unidirectional in a direction essentially perpendicular to the compressible surface (22), respectively the tissue surface (30).

8. Monitoring device according to one of the preceding claims, wherein the cushion structure (2) is releasably attached to the support structure (1).

9. Monitoring device according to one of the preceding claims, wherein the cushion structure (2) comprises a plurality of flexible panel elements (26) extending with one end from the contacting side (21) and carrying contact faces (23) at their other ends for contacting the tissue surface (30).

10. Monitoring device according to the preceding claim, wherein the plurality of flexible panel elements (26) in a relaxed state have a curved shape.

11. Monitoring device according to one of the preceding claims 9 or 10, wherein a pair of flexible panel elements (26) form an O-shape (27), wherein one side of the O-shape (27) is oriented towards the attachment side (20) and an opposing side of the O-shape (27) carries a contacting face (23).

12. Monitoring device according to one of the preceding claims, wherein an optical fiber (51) carrying a light emitter (50) is attached to a positioning sensor (5).

13. Monitoring device according to one of the preceding claims, wherein each positioning sensor (5) comprises a sensor body (11) and a sensor shaft (12) carrying a sensor tip (13) and being moveable relative to the sensor body (11), wherein the sensor shaft (12) is surrounded by a compressible sheath.

14. Probe unit (40) for a monitoring device for monitoring oxygenation, particularly for monitoring oxy- and deoxyhemoglobin concentration, oxidized and reduced cytochrome C oxidase, of a tissue (31) comprising a rigid support structure (1) and a cushion structure (2) according to any of the preceding claims.

15. Method for calibrating a monitoring device according to one of the claims 1 - 13, comprising a two-step calibration process, wherein in a first calibration step a linear function for determining surface measures is generated using the plurality of positioning sensors (5) on a set of defined calibration structures, and in a second calibration step the linear function is verified using the plurality of positioning sensors (5) on a set of defined tissue shape samples and/or surface shape samples.

16. Method for calibrating a monitoring device according to claim 15, wherein in the first calibration step the support structure (1) is used for the calibration measurements with the plurality of light emitters (50), the at least one light detector (10) and the plurality of positioning sensors (5) connected thereto and without an attached cushion structure, and in the second calibration step, the same support structure (1) with the plurality of light emitters (50), the at least one light detector (10) and the plurality of positioning sensors (5) connected thereto is used with an attached cushion structure (2).
